# EUROPÄISCHE PATENTSCHRIFT

(11) **EP 3 310 408 B1**
(45) Veröffentlichungstag und Bekanntmachung des Hinweises auf die Patenterteilung: **25.11.2020**
(21) Anmeldenummer: 16731509.2
(22) Anmeldetag: 15.06.2016
(51) Int. Cl.: A61P 7/08, A61M 1/28, A61K 33/00, C08B 31/04, C08B 31/06

(54) **DIALYSELÖSUNG, VERWENDUNG EINER DIALYSELÖSUNG SOWIE CHEMISCHE VERBINDUNG**
DIALYSIS SOLUTION, USE OF A DIALYSIS SOLUTION AND CHEMICAL COMPOUND
DIALYSAT, UTILISATION D'UN DIALYSAT ET CORPS COMPOSÉ

(30) Priorität: 16.06.2015 DE 102015007626
(43) Veröffentlichungstag der Anmeldung: 25.04.2018
(60) Teilanmeldung: 20201491.6
(73) Patentinhaber: Fresenius Medical Care Deutschland GmbH, 61352 Bad Homburg (DE); Friedrich-Schiller-Universität Jena, 07743 Jena (DE)
(72) Erfinder: BERLICH, Robert, 66606 St. Wendel (DE); SCHWEITZER, Thomas, 66589 Wemmetsweiler (DE); FINKLER, Lisa, 66620 Nonnweiler (DE); HEINZE, Thomas, 07743 Jena (DE); HAMPE, Robert, 07743 Jena (DE)
(74) Vertreter: Herrmann, Uwe
(86) Internationale Anmeldenummer: PCT/EP2016/000999
(87) Internationale Veröffentlichungsnummer: WO 2016/202455

(56) Entgegenhaltungen:
- US-A- 3 553 194
- US-A1- 2004 014 961
- US-B1- 6 248 726
- FEIGA R.T. ET AL.: "Note on the reaction of corn starch with chloromethylphosphonic dichloride in pyridine", CEREAL CHEMISTRY, Bd. 44, Nr. 5, September 1967 (1967-09), Seiten 554-557, XP002760389,
- HEINZE T. ET AL.: "Simple synthesis of mixed cellulose acylate phosphonates applying n-propyl phosphonic acid anhydride", CELLULOSE, Bd. 19, 2012, Seiten 523-531, XP002760390,

## Beschreibung

Die vorliegende Erfindung betrifft eine Dialyselösung mit wenigstens einem Osmotikum.

Dialyselösungen, wie sie beispielsweise bei der Peritonealdialyse, Hämodialyse, Hämodiafiltration etc. eingesetzt werden, sind in zahlreichen unterschiedlichen Zusammensetzungen bekannt.

So offenbart beispielsweise die DE 10 2004 023 828 A1 eine Lösung für eine Peritonealdialyse, die neben Elektrolyten ein Osmotikum in Form von Glucose enthält.

Die US 6,248,726 B1 offenbart eine Dialyselösung, die als Osmotikum eine lösliche abgebaute Stärke mit einem mittleren Molekulargewicht von etwa 3000 Dalton aufweist.

Die als Osmotikum dienende Glucose führt dazu, dass der Transport von Wasser über die Membran beschleunigt und somit die Ultrafiltrationsrate verbessert wird. Ein weiteres bekanntes Osmotikum ist Icodextrin, bei dem es sich um ein stärkebasiertes, verzweigtes Glucosepolymer handelt.

Der vorliegenden Erfindung liegt die Aufgabe zugrunde, eine Dialyselösung dahingehend weiterzubilden, dass deren Ultrafiltrationsleistung gegenüber bekannten Dialyselösungen gesteigert ist.

Diese Aufgabe wird durch eine Dialyselösung mit den Merkmalen des Anspruchs 1 gelöst. Danach ist vorgesehen, dass es sich bei dem Osmotikum um Stärkealkylphosphonat und insbesondere um Stärkepropylphosphonat oder Stärkeethylphosphonat handelt.

Stärkepropylphosphonate werden beispielsweise durch Reaktion von Stärke und Propylphosphonsäureanhydrid hergestellt, jedoch ist die Erfindung nicht auf diesen Weg der Synthese von Stärkepropylphosphonaten beschränkt.

Von der Erfindung sind auch weitere Stärkealkylphosphonate umfasst, zum Beispiel Stärkeethylphosphonate, welche unter anderem durch Umsetzung von Stärke mit Ethylphosphonsäureanhydrid erhalten werden können.

Es hat sich überraschenderweise gezeigt, dass Stärkealklyphosphonate eine sehr hohe Wirksamkeit bei der Verwendung als osmotische Agenzien aufweisen.

An dieser Stelle wird darauf hingewiesen, dass die Begriffe "Stärkealkylphosphonat", "Stärkepropylphosphonat", "Stärkeethylphosphonat" etc. im Rahmen der vorliegenden Erfindung diese Substanzen als solche sowie auch deren Salz bezeichnen können. Ein Salz dieser Substanz ist vorzugsweise ein pharmazeutisch akzeptables Salz, insbesondere das Natriumsalz.

In einer Ausgestaltung der Erfindung ist vorgesehen, dass die Dialyselösung genau eine Art oder auch mehrere Arten unterschiedlicher Stärkealkylphosphonate enthält. Diese mehreren Arten von Stärkealkylphosphonaten können sich beispielsweise im durchschnittlichen Substitutionsgrad und/oder auch in der molekularen Masse und/oder in dem Edukt unterscheiden, aus dem sie hergestellt sind.

In einer weiteren Ausgestaltung der Erfindung ist vorgesehen, dass die Dialyselösung außer Stärkealkylphosphonat kein weiteres Osmotikum enthält.

Von der Erfindung sind jedoch auch Ausführungsformen umfasst, bei denen außer Stärkealkylphosphonat ein oder mehrere weitere Osmotika, wie beispielsweise Glucose und/oder Icodextrin vorliegen.

Vorzugsweise ist das Stärkealkylphosphonat der erfindungsgemäßen Dialyselösung vollständig wasserlöslich.

Der durchschnittliche Substitutionsgrad des Stärkealkylphosphonats der erfindungsgemäßen Dialyselösung liegt vorzugsweise im Bereich von 0,1 bis 1,2.

Unter dem Begriff "Substitutionsgrad" wird im Rahmen der vorliegenden Erfindung die mittlere Anzahl von Substituenten pro Anhydroglucoseeinheit der Stärke verstanden. Der Substitutionsgrad kann Werte zwischen 0 und 3 annehmen.

Bevorzugt ist ein Bereich des durchschnittlichen Substitutionsgrades im Bereich von 0,2 bis 0,5.

Vorzugsweise ist das Stärkealkylphosphonat der erfindungsgemäßen Dialyselösung durch Umsetzung von Polysacchariden, insbesondere von Stärke und vorzugsweise von abgebauter Stärke hergestellt. Dabei weisen die Polysaccharide, insbesondere die Stärke eine zahlenmittlere Molmasse Mn vorzugsweise im Bereich von 1.000 g/mol - 50.000 g/mol, weiter vorzugsweise im Bereich von 1.000 g/mol - 20.000 g/mol und besonders bevorzugt im Bereich von 3.000 g/mol - 5.000 g/mol auf.

In einer weiteren bevorzugten Ausgestaltung der Erfindung erfolgt die Reduktion der Stärke mit Natriumborhydrid vor der Umsetzung zum Stärke-n-propylphosphonat bzw. zum Stärkealkylphosphonat bzw. Stärke-n-alkylphosphonat. Die Behandlung der Stärke (Reduktion) kann über die Verwendung von Natriumborhydrid hinaus auch mit weiteren Hydridübertragungsreagenzien wie Tetraalkylammoniumborhydrid, Natriumcyanoborhydrid, Lithiumcyanoborhydrid oder Boran-Amin-komplexen (z.B. Boran-t-butylamin-Komplex) erfolgen.

Gemäß einem Verfahren zur Herstellung des erfindungsgemäßen Osmotikums kann somit die Stärke reduziert werden, bevor sie zu dem Stärkealkylphosphonat umgesetzt wird.

Ein Verfahren zur Herstellung des erfindungsgemäßen Osmotikums kann einen oder mehrere der im Rahmen dieser Erfindung offenbarten Verfahrensschritte umfassen.

Die Reduktion des Ausgangsmaterials führt nach Substitution und Sterilisierung zu annähernd farblosen Proben, während die bisher synthetisierten (unreduzierten) Proben eine Braunfärbung aufwiesen, die auf bisher unbekannte Nebenprodukte zurückzuführen ist. Von der Performance her findet sich kein Unterschied zwischen reduzierten und unreduzierten Proben.

Vorzugsweise enthält die Dialyselösung außer dem oder den Osmotika Elektrolyte sowie wenigstens einen Puffer.

Bei den Elektrolyten kann es sich um Ionen von Natrium und/oder Kalium und/oder Calcium und/oder Magnesium handeln. Als Anion ist vorzugsweise Chlorid enthalten.

Der oder die Puffer können Laktationen und/oder Hydrogencarbonationen aufweisen. Der Puffer dient zur Einstellung eines physiologischen pH-Wertes. Der pH-Wert der Dialyselösung liegt vorzugsweise im Bereich zwischen 5,0 und 8,0. Besonders bevorzugt liegt der pH-Wert der Dialyselösung im Bereich von 6,8 bis 7,2.

Handelt es sich um ein Einkammerbeutelsystem liegt der pH-Wert vorzugsweise zwischen 5,0 und 8,0, besonders bevorzugt zwischen 5,5 und 6,5. Handelt es sich um ein Zweikammerbeutelsystem liegt der Misch pH-Wert (nach dem Mischen der - Teillösungen) vorzugsweise zwischen 5,0 und 8,0, und besonders bevorzugt zwischen 6,5 und 7,5. Der pH-Wert der sauren Teillösung liegt vorzugsweise zwischen 3,0 und 5,0 und der pH-Wert der basischen Teillösung liegt vorzugsweise zwischen 7,0 und 9,0.

In einer Ausführungsform liegen die Elektrolyte, soweit vorhanden und unabhängig voneinander, in den folgenden Konzentrationen in der Dialyselösung vor (Angaben in mmol/l):
Natriumionen 125-150
Kaliumionen 0-4,5
Calciumionen 0-2,5
Magnesiumionen 0-5
Chloridionen 90-120
Lactat/Milchsäure/(Hydrogen)Carbonat/CO2 30-60

Die erfindungsgemäße Dialyselösung kann beispielsweise bei der Hämodialyse, bei der Hämodiafiltration oder auch bei der Peritonealdialyse eingesetzt werden.

Die vorliegende Erfindung betrifft des Weiteren die erfindungsgemäße Dialyselösung zur Verwendung in einem Blutbehandlungsverfahren.

Wie ausgeführt, kann es sich bei dem Blutbehandlungsverfahren um die Hämodialyse oder auch um die Hämodiafiltration handeln.

Auch ist der Einsatz der erfindungsgemäßen Dialyselösung bei der Peritonealdialyse denkbar und von der Erfindung mit umfasst.

Vorzugsweise wird das Stärkealkylphosphonat, das in der erfindungsgemäßen Dialyselösung Verwendung findet, aus einem oder mehreren Polysacchariden und insbesondere aus Stärke nach einem an sich bekannten Verfahren hergestellt. Dabei können Polysaccharide, insbesondere Stärke mit unterschiedlichen molekularen Massen und insbesondere mit vergleichsweise geringen molekularen Massen im Bereich von 1.000 g/mol - 50.000 g/mol, weiter vorzugsweise im Bereich von 1.000 g/mol - 20.000 g/mol und besonders bevorzugt im Bereich von 3.000 g/mol - 5.000 g/mol verwendet werden.

Vorzugsweise weisen die bevorzugt vollständig wasserlöslichen Stärkealkylphosphonate gemäß der Erfindung einen durchschnittlichen Substitutionsgrad (DS) im Bereich von 0,1 bis 1,2 und insbesondere im Bereich von 0,2 bis 0,5 auf.

Wie dies aus den im Folgenden dargestellten Ausführungsbeispielen hervorgeht, zeigen Stärkealkylphosphonate bei der Verwendung als osmotische Agenzien eine hervorragende Wirksamkeit.

Unter dem Begriff "Substitutionsgrad" wird im Rahmen der vorliegenden Erfindung die mittlere Anzahl von Substituenten pro Anhydroglucoseeinheit der Stärke verstanden. Der Substitutionsgrad kann Werte zwischen 0 und 3 annehmen.

Vorzugsweise ist das im Rahmen der vorliegenden Erfindung eingesetzte Stärkealkylphosphonat durch Umsetzung von Polysacchariden, insbesondere von Stärke und vorzugsweise von abgebauter Stärke hergestellt. Dabei weisen die Polysaccharide, insbesondere die Stärke eine zahlenmittlere Molmasse Mn vorzugsweise im Bereich von 1.000 g/mol - 50.000 g/mol, weiter vorzugsweise im Bereich von 1.000 g/mol - 20.000 g/mol und besonders bevorzugt im Bereich von 3.000 g/mol - 5.000 g/mol auf.

Vorzugsweise ist das Stärkepropylphosphonat aus den sich wiederholenden Einheiten 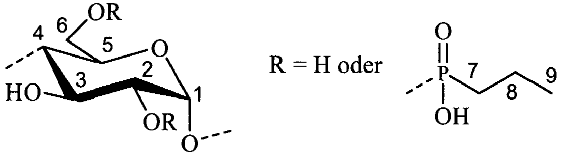 aufgebaut bzw. enhält diese sich wiederholenden Einheiten. Statt R=H sind auch andere Substituenten denkbar und von der Erfindung mit umfasst.

Die Substitution mit Propylphosphonat ist vorzugsweise an den Positionen 6, 2 und 3 der Glucoseeinheiten möglich, wobei überwiegend die Positionen 6 und 2 substituiert werden. Durch die Wahl der Reaktionsbedingungen ist es weiterhin möglich, eine bevorzugte Substitution an Position 6 oder 2 zu erreichen. Bei den in den Beispielen 1 bis 4 wiedergegebenen homogenen Reaktionsbedingungen erfolgt die Substitution überwiegend an Position 6. Durch heterogene Reaktionsbedingungen z.B. mit NaOH ist eine bevorzugte Substitution an der Position 2 möglich.

Die Anzahl der Propylphosphonateinheiten bzw. Alkylphosphonateinheiten pro Anhydroglucoseeinheit ist der im Rahmen der vorliegenden Erfindung genannte Substitutionsgrad.

Als Ausgangsmaterialien für die Herstellung des Stärkealkylphosphonats sind Stärken verschiedener Quelle (z.B. aus Kartoffel, Mais, Maniok (Tapioka), Reis, Erbse, Weizen und weitere Getreidearten) sowie spezielle Stärketypen wie Hylon VII, Amiocapowder oder Wachsmaisstärke denkbar. Die vorstehende Aufzählung ist exemplarisch und nicht abschließend.

Weitere Einzelheiten und Vorteile werden anhand eines in der Zeichnung dargestellten Ausführungsbeispiels näher erläutert.

Es zeigen:
- Figur 1:: Schematische Darstellungen eines mit der erfindungsgemäßen Dialyselösung gefüllten Schlauches zu unterschiedlichen Zeitpunkten,
- Figur 2:: Die Zunahme des Volumens eines mit einer Dialyselösung gefüllten Schlauches für verschiedene Osmotika über die Zeit und
- Figur 3:: Die Volumenzunahme des Schlauches nach 24 Stunden Einwirkzeit für verschiedene Osmotika,
- Figuren 4 bis 15:: ¹H-NMR und ¹³C-NMR Spektren von unterschiedlichen Stärkepropylphosphonaten und Stärkeethylphosphonaten.

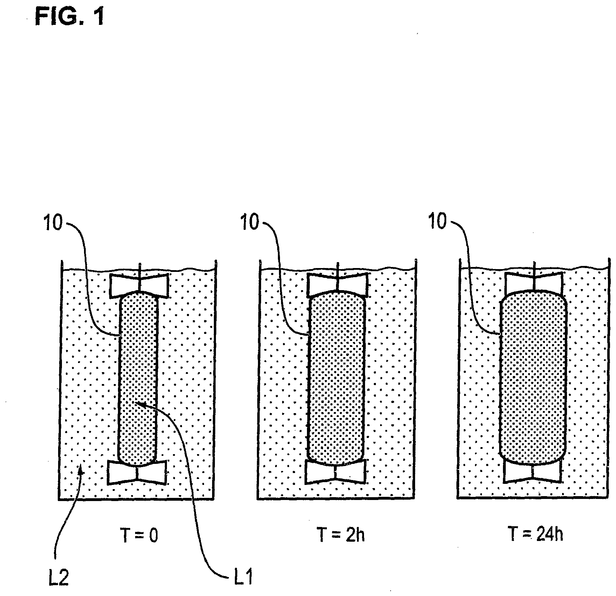

Figur 1 zeigt einen Schlauch 10, dessen Wandung durch eine semipermeable Membran gebildet wird. Bei der Membran kann es sich beispielsweise um Cellulose bzw. um regenerierte Cellulose handeln.

In dem Inneren des Schlauches 10 befindet sich eine Dialyselösung L1, die ein Osmotikum enthält. Der Schlauch 10 befindet sich in einer Lösung L2, die dieselbe Zusammensetzung aufweist wie die Lösung L1 in dem Schlauch 10 mit dem einzigen Unterschied, dass die Lösung L2 kein Osmotikum aufweist.

Figur 1, linke Darstellung zeigt die Anordnung zum Zeitpunkt T=0, d.h. zu dem Versuchsbeginn, zu dem der gefüllte Schlauch 10 in die Lösung L2 eingesetzt wurde.

Figur 1, mittlere Darstellung zeigt die Anordnung nach zweistündiger Einwirkdauer (T=2 h) und Figur 1, rechte Darstellung zeigt die Anordnung nach 24-stündiger Einwirkdauer (T=24 h).

Wie dies aus einem Vergleich der Abbildungen gemäß Figur 1 hervorgeht, ist aufgrund der osmotischen Wirkung der Lösung L1 Wasser in den Schlauch eingeströmt, womit sich dessen Volumen über die Zeit entsprechend vergrößert.

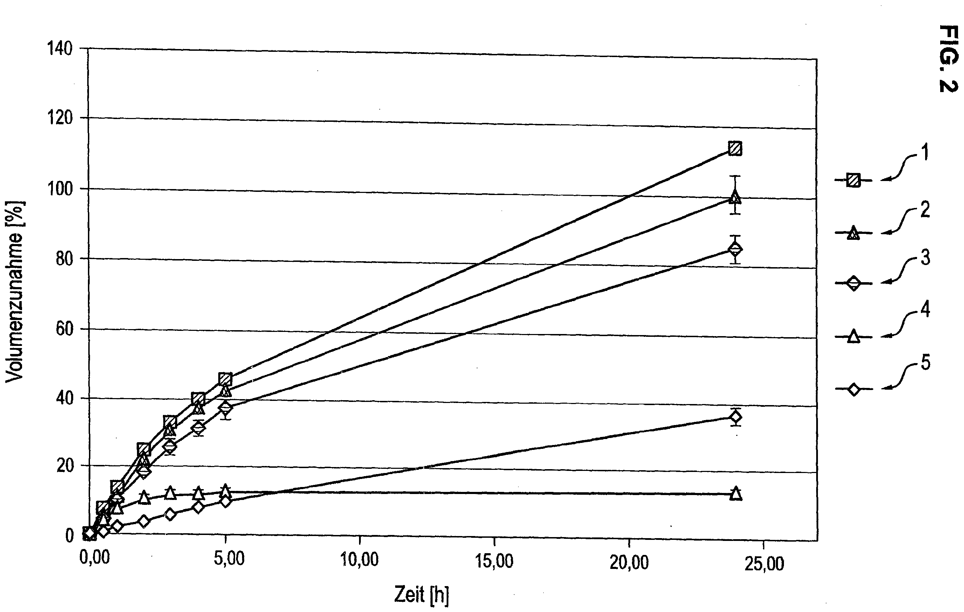

Figur 2 zeigt die Volumenzunahme in % (ausgehend von dem Versuchsstart bei T=0) über die Zeit bei einem weiteren Schlauchversuch für Lösungen mit unterschiedlichen Osmotika. Dabei weisen die Lösungen 1, 2 und 3 Stärkepropylphosphonate mit einem durchschnittlichen Substitutionsgrad DS von 1,19 (Lösung 1), DS von 0,62 (Lösung 2) und DS von 0,23 (Lösung 3) auf.

Die Bezugszeichen 4 und 5 betreffen Lösungen mit Glucose (Lösung 4) und mit Icodextrin (Lösung 5).

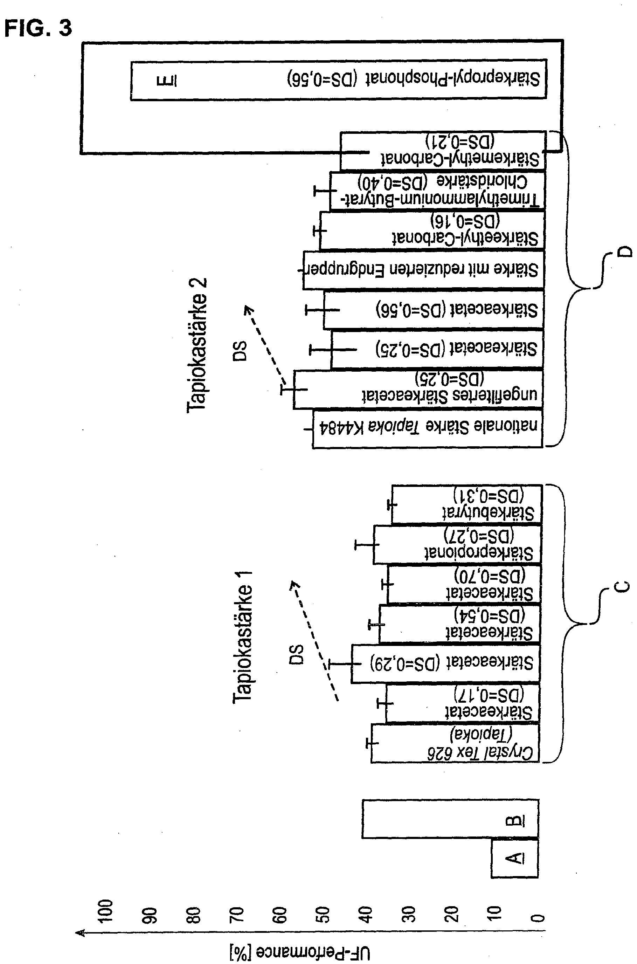

Die Auswertung der Ergebnisse für unterschiedliche Osmotika ist in Figur 3 gezeigt, wobei Figur 3 auf der Ordinate den Volumenzuwachs, d.h. die UF-Performance (UF=Ultrafiltration) des Schlauches 10 nach 24-stündiger Einwirkdauer zeigt. Der Wert 0 % bedeutet, dass sich gegenüber dem Versuchsbeginn (T=0) keine Volumenänderung ergeben hat, der Wert 100 % bedeutet gegenüber dem Ausgangszustand bei T=0 eine Volumenverdopplung.

Die Versuchsbedingungen, die den Ergebnissen gemäß Figur 2 und Figur 3 zugrunde liegen, waren für beide Figuren 2 und 3 identisch.

Das Bezugszeichen A zeigt das Ergebnis bei dem Einsatz von Glucose und verdeutlicht, dass nach 24-stündiger Einwirkzeit eine Volumenzunahme um 10% erfolgt ist. Das Bezugszeichen B zeigt das Ergebnis bei der Verwendung einer Icodextrinlösung, die nach 24-stündiger Einwirkdauer eine Volumenzunahme von 40% bewirkt.

Das Bezugszeichen C kennzeichnet Lösungen mit unterschiedlichen Stärken bzw. Stärkederivaten basierend auf einer Tapiokastärke mit einer zahlenmittleren Molmasse von 4.898 g/mol (in Figur 3 als "Tapiokastärke 1" bezeichnet) und das Bezugszeichen D zeigt Lösungen mit unterschiedlichen Stärken und Stärkederviaten basierend auf einer Tapiokastärke mit einer zahlenmittleren Molmasse von 3.321 g/mol (in Figur 3 als "Tapiokastärke 2" bezeichnet). Die verwendeten Stärken der Gruppen C und D sind jeweils mit steigenden durchschnittlichen Substitutionsgraden (DS) von links nach rechts wiedergegeben. Der durch den Einsatz dieser Osmotika bedingte Volumenzuwachs liegt in etwa in der Spanne 35 % bis 55 %.

Das Bezugszeichen E zeigt eine Dialyselösung gemäß der vorliegenden Erfindung, die Stärkepropylphosphonat enthält und zwar mit einem durchschnittlichen Substitutionsgrad (DS) von 0,54.

Aus Figur 3 wird deutlich, dass bei dem Einsatz der erfindungsgmäßen Osmotika, d.h. bei dem Einsatz von Stärkepropylphosphonaten ein Volumenzuwachs von über 90% erzielt wird, d.h. ein Volumenzuwachs, der deutlich über dem liegt, der mit bekannten Osmotika erreicht wird.

Der Gehalt (Gew.-%) an Osmotikum aller in Figur 3 gezeigten Lösungen sowie deren sonstige Zusammensetzung ist identisch.

Die Versuchsbedingungen für die Ergebnisse gemäß Figur 2 und 3 sind wie folgt:
In einen Schlauch mit einer semipermeablen Schlauchwandung aus regenerierter Cellulose (MWCO: 1000 Da, Fa. Roth) wurde ein Füllvolumen von 10 ml einer Flüssigkeit eingefüllt. Diese Flüssigkeit besteht aus einer wässrigen Lösung eines osmotischen Agens (Stärkepropylphosphonat, Glucose, Icodextrin, Stärke oder Stärkederivat) mit einer Konzentration des Osmotikums von 5 Gew.-%, wobei weitere Inhaltsstoffe durch Ca²⁺ in einer Konzentration von 1 mmol/l, Mg²⁺ in einer Konzentration von 0,5 mmol/l, Na⁺ in einer Konzentration von 138 mmol/l, Cl⁻ in einer Konzentration von 106 mmol/l und Lactat in einer Konzentration von 35 mmol/l vorliegen.

Dieser gefüllte Schlauch wurde bei einer Temperatur von 38 °C in einem Bad derselben Versuchslösung allerdings ohne osmotisches Agens für 24 Stunden unter Bewegung gelagert.

Zu verschiedenen Zeitpunkten wurde die Volumenzunahme des Füllvolumens des Schlauches ermittelt, die die osmotische Wirkung des Agens wiederspiegelt. Wie dies aus Figur 2 ersichtlich ist, wurden die osmotischen Agenzien gemäß der vorliegenden Erfindung mit bekanntem osmotischen Agenzien in Form von Glucose und Icodextrin verglichen.

Figur 2 zeigt, dass nach 24-stündiger Einwirkdauer die Volumenzunahme des Schlauches für sämtliche Dialyselösungen, die Stärkepropylphosphonate enthalten über 80 % liegt. Als Maximalwert wird eine Volumenzunahme von ca. 115 % nach 24 Stunden erreicht.

Demgegenüber liegen die Endwerte nach 24 h für Icodextrin bei ca. 35 % und von Glucose bei ca. 13 %.

Die erfindungsgemäßen Osmotika zeigen nicht nur eine erhöhte Ultrafiltrationseffizienz nach 24 Stunden, sondern gegenüber Icodextrin auch einen höheren Wert bei geringen Verweildauern.

Während die Volumenzunahme bei Icodextrin im Wesentlichen linear verläuft, ist bei den Dialyselösungen, die Stärkepropylphosphonate enthalten, ein vergleichsweise steiler Anstieg zu erkennen, der dann, bei höheren Verweilzeiten etwas abflacht und in einen im Wesentlichen linearen Verlauf übergeht.

Der Anstieg des Schlauchvolumens bei kleinen Verweilzeiten ist bei dem Einsatz von Stärkepropylphosphonaten vergleichbar mit dem von Glucose. Bei höheren Werten ist jedoch der Volumenanstieg bei Glucose als Osmotikum weitaus geringer und bleibt ab einer Verweildauer von ca. drei Stunden konstant, wie dies aus Figur 2 hervorgeht.

Die Stärkepropylphosphonate gemäß der Lösungen 1, 2 und 3 wurden aus Tapiokastärke mit einer zahlenmittleren Molmasse von 3.321 g/mol hergestellt.

Die zahlenmittlere Molmasse ist im Rahmen der vorliegenden Erfindung mit Mn angegeben.

Im Folgenden werden Ausführungsbeispiele zur Herstellung von Stärkepropylphosphonat beschrieben. Bis auf die jeweils genannten Unterschiede waren die Herstellbedingungen für alle vier im Folgenden aufgeführten Beispiele identisch:

### Beispiel 1:

40,0 g abgebaute Tapiokastärke (Mn= 3.321 g/mol) werden in 360 ml N,N-Dimethylformamid (DMF) suspendiert und unter Stickstoffatmosphäre bei 120 °C 2 h unter Rühren behandelt. Nach dem Abkühlen auf 80 °C werden 14 g LiCI zugefügt und unter weiterem Rühren löst sich die Stärke nach ca. 1 h auf.

Die Lösung wird auf 100 °C erhitzt und 23,6 g (0,3 mol/mol Anhydroglucoseeinheit, AGE) Propylphosphonsäureanhydrid (T3P) in DMF (51,5 %, w/w) zugegeben. Nach 3 h Reaktionszeit wird das Produkt in 3 l Ethanol gefällt, filtriert, dreimal mit 1 l Ethanol gewaschen und aus 70 ml Wasser in 1 I Ethanol umgefällt.

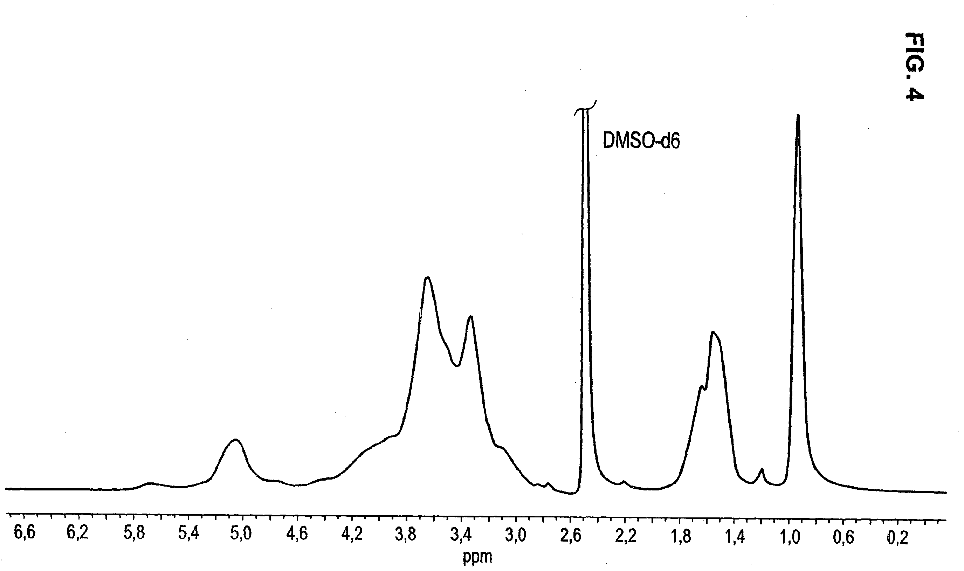

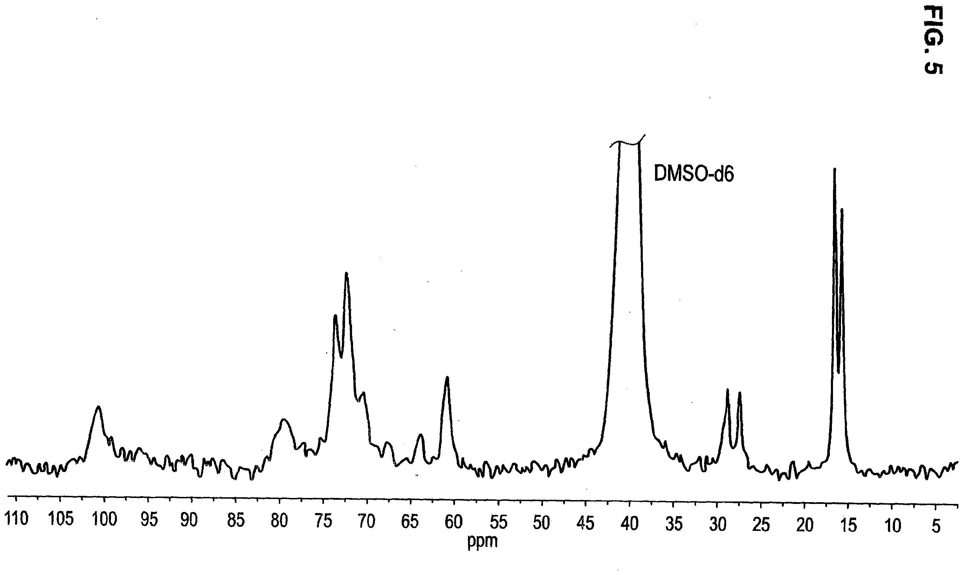

Das Produkt wird in 400 ml Wasser gelöst, die Lösung mit Amberlite IR120 (H⁺-Form) behandelt, der Ionenaustauscher abgetrennt und die Polymerlösung gefriergetrocknet. Der Strukturbeleg erfolgte durch ¹H- und ¹³C-NMR-Spektren (Figuren 4 und 5).

Durchschnittlicher Substitutionsgrad DS (bestimmt mittels ¹H-NMR-Spektroskopie): 0,54.

Figur 4 zeigt das ¹H-NMR-Spektrum von Stärkepropylphosphonat, aufgenommen in DMSO-d6 versetzt mit Trifluoressigsäure und Figur 5 zeigt das ¹³C-NMR-Spektrum von Stärkepropylphosphonat, aufgenommen in DMSO-d6.

### Beispiel 2:

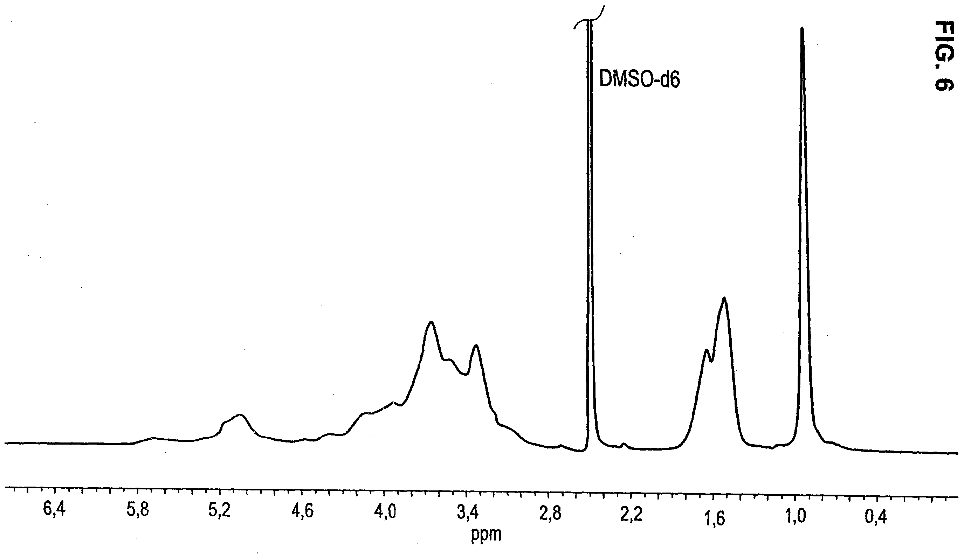

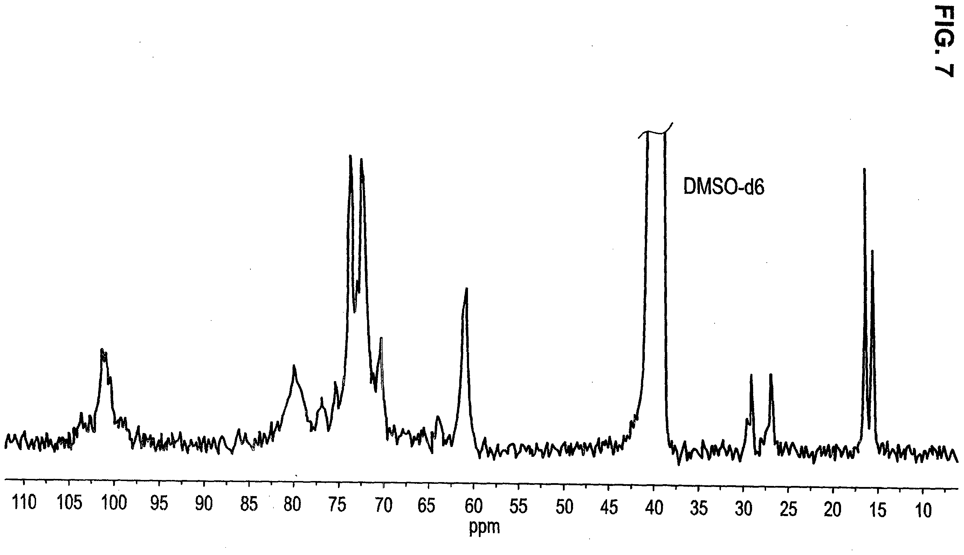

Gemäß Beispiel 1 wird abgebaute Tapiokastärke mit einer Molmasse (Mn) von 4.898 g/mol unter identischen Bedingungen wie in Beispiel 1 umgesetzt. Das Fällen und Umfällen aus wässriger Lösung erfolgen mit Aceton. ¹H- und ¹³C-NMR-Spektren bestätigen die Struktur (Figuren 6 und 7).

DS (bestimmt mittels ¹H-NMR-Spektroskopie): 0,70.

Figur 6 zeigt das ¹H-NMR-Spektrum von Stärkepropylphosphonat, aufgenommen in DMSO-d6 versetzt mit Trifluoressigsäure und Figur 7 zeigt das ¹³C-NMR-Spektrum von Stärkepropylphosphonat, aufgenommen in DMSO-d6.

### Beispiel 3:

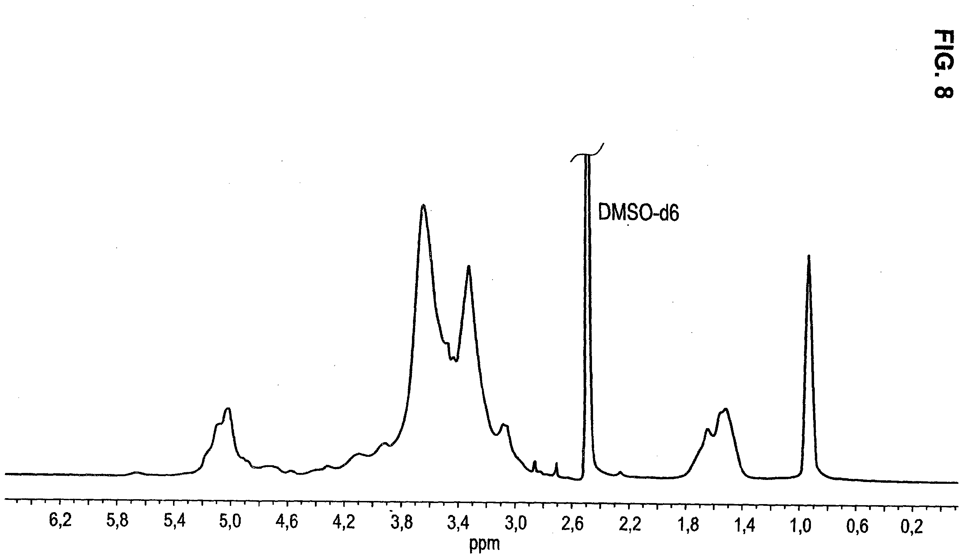

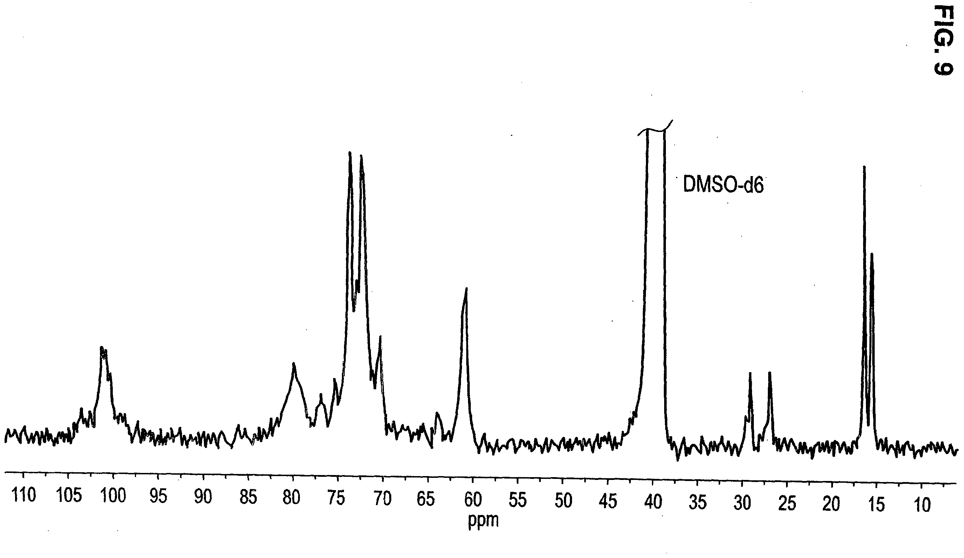

Gemäß Beispiel 1 werden 40,0 g abgebaute Tapiokastärke (Mn = 3.321g/mol) mit 7,9 g (0,1 mol/mol AGE) T3P in DMF (50 %, w/w) umgesetzt. Das Stärkederivat wird durch Fällung und Umfällung aus Wasser mit Isopropanol isoliert und gereinigt. ¹H- und ¹³C-NMRSpektren bestätigen die Struktur (Figuren 8 und 9).

DS (bestimmt mittels 1H-NMR-Spektroskopie): 0,23.

Figur 8 zeigt das ¹H-NMR-Spektrum von Stärkepropylphosphonat, aufgenommen in DMSO-d6 versetzt mit Trifluoressigsäure und Figur 9 zeigt das ¹³C-NMR-Spektrum von Stärkepropylphosphonat, aufgenommen in DMSO-d6.

### Beispiel 4:

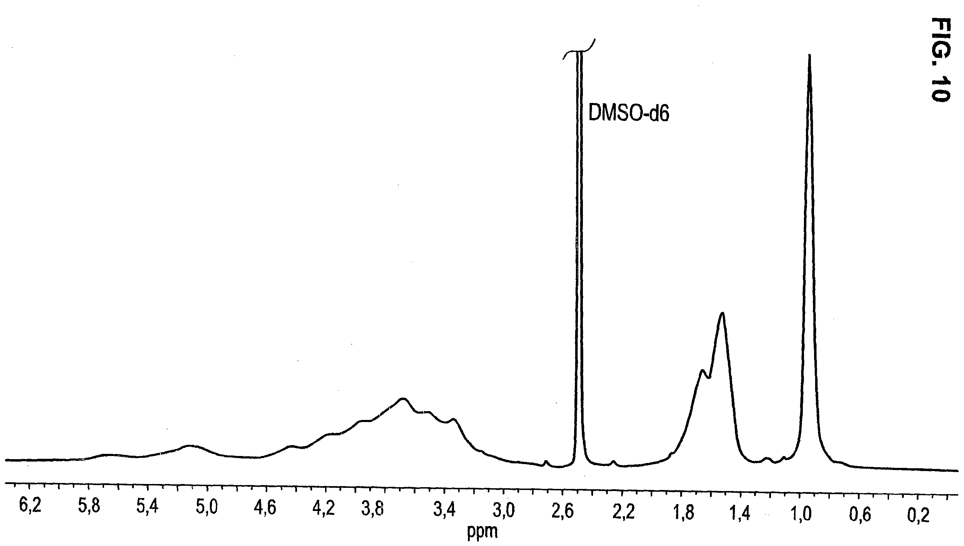

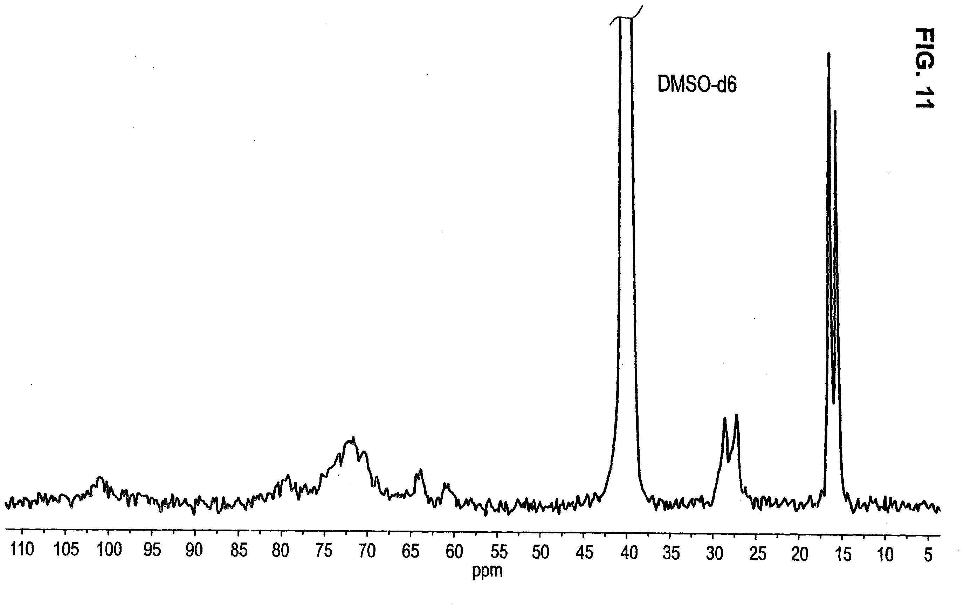

Gemäß Beispiel 1 werden 40,0 g abgebaute Tapiokastärke (Mn= 3.321 g/mol) mit 47,1 g (0,6 mol/mol AGE) T3P in DMF (50 %, w/w) umgesetzt. Das Stärkederivat wird durch Fällung und Umfällung aus Wasser mit Isopropanol isoliert und gereinigt. ¹H- und ¹³C-NMR Spektren bestätigen die Struktur (Figuren 10 und 11). DS (bestimmt mittels ¹H-NMR-Spektroskopie): 1,19.

Figur 10 zeigt das ¹H-NMR-Spektrum von Stärkepropylphosphonat, aufgenommen in DMSO-d6 versetzt mit Trifluoressigsäure und Figur 11 zeigt das ¹³C-NMR-Spektrum von Stärkepropylphosphonat, aufgenommen in DMSO-d6.

### Beispiel 5:

60 g Tapioastärke (Mn = 3.321g/mol) werden in 150 mL Wasser bei 80 °C gelöst und die Lösung mit Natriumhydrogencarbonat auf einen pH-Wert von 8 eingestellt. Nach Abkühlung auf 60 °C wird portionsweise 1,37 g Natriumborhydrid (2 mol/mol reduzierende Endgruppe) zugefügt und lässt das Reaktionsgemisch 24 h unter Rühren reagieren. Nach dem Abkühlen auf Raumtemperatur wird die Lösung mit 15%iger Salzsäure auf einen pH-Wert von 6 eingestellt und 1 h gerührt. Das Produkt wird in 1,5 L Ethanol gefällt, filtriert, drei Mal mit 500 mL Ethanol gewaschen und im Vakuum bei 40 °C getrocknet. Mit Hilfe des Fehlingtests sind keine Carbonylgruppen nachweisbar.

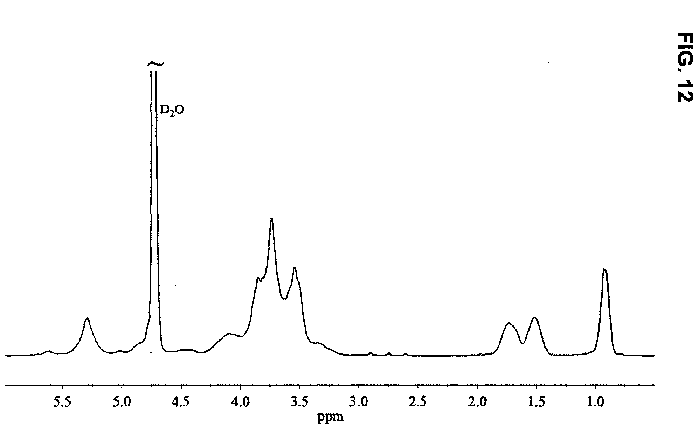

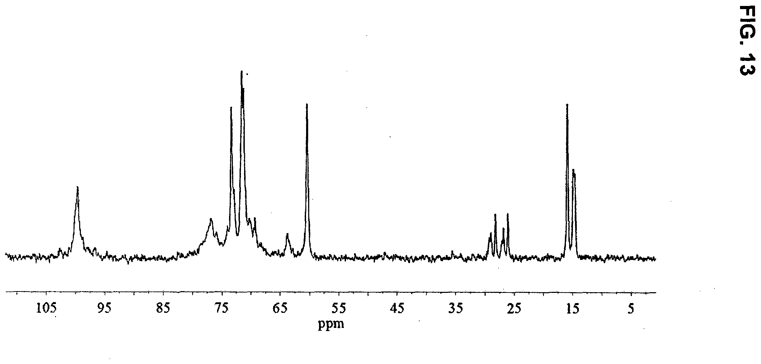

Gemäß Beispiel 1 werden 20,0 g der mit Natriumborhydrid behandelten Stärke mit 11,8 g (0,3 mol/mol AGE) T3P in DMF (50 %, w/w) umgesetzt. Das Produkt wird durch Fällung in Ethanol und Umfällung aus Wasser in Ethanol isoliert und gereinigt, in 400 mL Wasser gelöst, die Lösung mit Amberlite IR120 (H⁺ -Form) behandelt und gefriergetrocknet. ¹H- und ¹³C-NMR-Spektren bestätigen die Struktur (Abbildungen 12 und 13).

1 DS (bestimmt mittels H-NMR-Spektroskopie): 0,39.

Figur 12 zeigt das ¹H-NMR-Spektrum von Stärkepropylphosphonat aufgenommen in D₂O und Figur 13 zeigt das ¹³C-NMR-Spektrum von Stärkepropylphosphonat, aufgenommen in D₂O.

### Beispiel 6

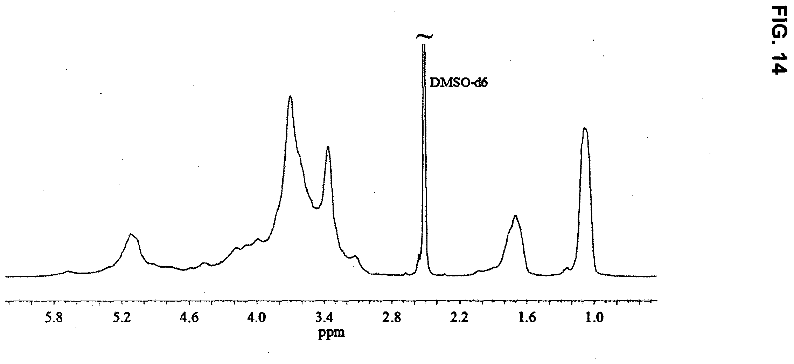

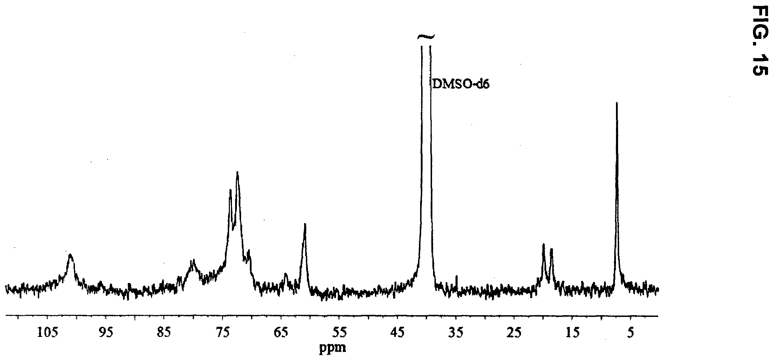

1,0 g der gemäß Beispiel 5 mit Natriumborhydrid behandelten Stärke werden bei 120 °C in 9 mL N,N-Dimethylacetamid (DMAc) unter Rühren gelöst. Nach Abkühlen auf 100 °C werden 0,51 g (0,3 mol/mol AGE) Ethylphosphonsäureanhydrid gelöst in Ethylacetat (66 %, w/w) zugegeben. Nach 3 h Reaktionszeit wird das Produkt in 100 mL Isopropanol gefällt, filtriert, dreimal mit 100 mL Isopropanol gewaschen und aus 5 mL Wasser in 50 mL Isopropanol umgefällt. Das Produkt wird in 50 mL Wasser gelöst und die Polymerlösung gefriergetrocknet. ¹H- und ¹³C-NMR-Spektren bestätigen die Struktur (Abbildungen 14 und 15).

DS (bestimmt mittels ¹H-NMR-Spektroskopie): 0,43.

Im Synthesebeispiel 6 wurde eine neue Lösungsmittelkombination (DMAc + Ethylacetat) verwendet, die im Vergleich zum bisher verwendeten DMF zu einem Produkt mit verbesserter Reinheit führt. Dies wurde auch bei der Synthese der Stärkepropylphosphonate beobachtet.

Figur 14 zeigt das ¹H-NMR-Spektrum von Stärkeethylphosphonat aufgenommen in DMSO-d6 versetzt mit Trifluoressigsäure und Figur 15 zeigt das ¹³C-NMR-Spektrum von Stärkeethylphosphonat aufgenommen in DMSO-d6.

Durch die vorliegende Erfindung wird eine Dialyselösung bereitgestellt, die gegenüber bekannten Dialyselösungen eine erheblich erhöhte osmotische Aktivität aufweist, was auf den Einsatz von Stärkealkylphosphonat als Osmotikum zurückzuführen ist.

Damit eignet sich die Dialyselösung beispielsweise als Peritonealdialyselösung oder auch im Rahmen der Hämodialyse, Hämodiafiltration, Hämofiltration oder auch im Rahmen sonstiger Blutbehandlungsverfahren, bei denen eine Behandlungslösung in Form der erfindungsgemäßen Dialyselösung oder als Substitutionslösung zum Einsatz kommt.

## Patentansprüche

1. Dialyselösung mit wenigstens einem Osmotikum,
**dadurch gekennzeichnet,**
**dass** es sich bei dem Osmotikum um Stärkealkylphosphonat und insbesondere um Stärkepropylphosphonat und/oder Stärkeethylphosphonat handelt.

2. Dialyselösung nach Anspruch 1, **dadurch gekennzeichnet, dass** die Dialyselösung genau eine Art eines Stärkealkylphosphonates oder mehrere Arten von Stärkealkylphosphonaten enthält.

3. Dialyselösung nach Anspruch 1 oder 2, **dadurch gekennzeichnet, dass** die Dialyselösung außer Stärkealkylphosphonat kein weiteres Osmotikum enthält.

4. Dialyselösung nach einem der vorhergehenden Ansprüche, **dadurch gekennzeichnet, dass** das Stärkealkylphosphonat vollständig wasserlöslich ist und/oder dass das Stärkealkylphosphonat aus reduzierter Stärke gebildet ist.

5. Dialyselösung nach einem der vorhergehenden Ansprüche, **dadurch gekennzeichnet, dass** das Stärkealkylphosphonat einen durchschnittlichen Substitutionsgrad im Bereich von 0,1 bis 1,2 aufweist.

6. Dialyselösung nach Anspruch 5, **dadurch gekennzeichnet, dass** das Stärkealkylphosphonat einen durchschnittlichen Substitutionsgrad im Bereich von 0,2 bis 0,5 aufweist.

7. Dialyselösung nach einem der vorhergehenden Ansprüche, **dadurch gekennzeichnet, dass** das Stärkealkylphosphonat aus einem Polysaccharid, vorzugsweise aus abgebauter Stärke mit einem mittleren Molekulargewicht Mn im Bereich von 1.000-50.000 g/mol, vorzugsweise 1.000-20.000 g/mol und besonders bevorzugt 3.000-5.000 g/mol hergestellt ist.

8. Dialyselösung nach einem der vorhergehenden Ansprüche, **dadurch gekennzeichnet, dass** die Lösung Elektrolyte sowie einen Puffer aufweist.

9. Dialyselösung nach Anspruch 8, **dadurch gekennzeichnet, dass** die Elektrolyte die Ionen von Natrium und/oder Kalium und/oder Calcium und/oder Magnesium umfassen.

10. Dialyselösung nach Anspruch 8 oder 9, **dadurch gekennzeichnet, dass** der Puffer Lactationen und/oder Hydrogencarbonationen aufweist.

11. Dialyselösung gemäß einem der Ansprüche 1 bis 10 zur Verwendung in einem Dialyseverfahren.

12. Dialyselösung nach Anspruch 11, **dadurch gekennzeichnet, dass** es sich bei dem Dialyseverfahren um die Hämodialyse oder um die Hämodiafiltration handelt.

13. Dialyselösung nach Anspruch 11, **dadurch gekennzeichnet, dass** es sich bei dem Dialyseverfahren um die Peritonealdialyse handelt.

## Claims

1. A dialysis solution having at least one osmotic agent,
**characterised in that**
the osmotic agent is starch alkylphosphonate and in particular starch propylphosphonate and/or starch ethylphosphonate

2. The dialysis solution according to claim 1, **characterised in that** the dialysis solution contains exactly one kind of starch alkylphosphonate or a plurality of kinds of starch alkylphosphonates.

3. The dialysis solution according to claim 1 or 2, **characterised in that** the dialysis solution does not contain any further osmotic agent except for starch alkylphosphonate.

4. The dialysis solution according to one of the preceding claims, **characterised in that** the starch alkylphosphonate is completely water-soluble and/or that the starch alkylphosphonate is formed from reduced starch.

5. The dialysis solution according to one of the preceding claims, **characterised in that** the starch alkylphosphonate has an average degree of substitution in the range from 0.1 to 1.2.

6. The dialysis solution according to claim 5, **characterised in that** the starch alkylphosphonate has an average degree of substitution in the range from 0.2 to 0.5.

7. The dialysis solution according to one of the preceding claims, **characterised in that** the starch alkylphosphonate is produced from a polysaccharide, preferably from degraded starch having an average molecular weight Mn in the range from 1,000 - 50,000 g/mol, preferably 1,000 - 20,000 g/mol and particularly preferably 3,000 to 5,000 g/mol.

8. The dialysis solution according to one of the preceding claims, **characterised in that** the solution comprises electrolytes and a buffer.

9. The dialysis solution according to claim 8, **characterised in that** the electrolytes comprise the ions of sodium and/or potassium and/or calcium and/or magnesium.

10. The dialysis solution according to claim 8 or 9, **characterised in that** the buffer comprises lactate ions and/or hydrogencarbonate ions.

11. The dialysis solution according to one of claims 1 to 10 for use in a dialysis method.

12. The dialysis solution according to claim 11, **characterised in that** the dialysis method is haemodialysis or haemodiafiltration.

13. The dialysis solution according to claim 11, **characterised in that** the dialysis method is peritoneal dialysis.

## Revendications

1. Solution de dialyse avec au moins un agent osmotique,
**caractérisée en ce que**
l'agent osmotique est un alkylphosphonate d'amidon et en particulier un propylphosphonate d'amidon et/ou un éthylphosphonate d'amidon.

2. Solution de dialyse selon la revendication 1, **caractérisée en ce que** la solution de dialyse contient précisément une sorte d'un alkylphosphonate d'amidon ou plusieurs sortes d'alkylphosphonates d'amidon.

3. Solution de dialyse selon la revendication 1 ou 2, **caractérisée en ce que** la solution de dialyse ne contient outre l'alkylphosphonate d'amidon aucun autre agent osmotique.

4. Solution de dialyse selon l'une quelconque des revendications précédentes, **caractérisée en ce que** l'alkymphosphonate d'amidon est totalement soluble dans l'eau, et/ou **en ce que** l'alkylphosphonate d'amidon est obtenu à partir d'amidon réduit.

5. Solution de dialyse selon l'une quelconque des revendications précédentes, **caractérisée en ce que** l'alkylphosphonate d'amidon présente un degré de substitution moyen dans la plage de 0,1 à 1,2.

6. Solution de dialyse selon la revendication 5, **caractérisée en ce que** l'alkylphosphonate d'amidon présente un degré de substitution moyen dans la plage de 0,2 à 0,5.

7. Solution de dialyse selon l'une quelconque des revendications précédentes, **caractérisée en ce que** l'alkylphosphonate d'amidon est fabriquée à partir d'un polysaccharide, de préférence à partir d'amidon décomposé avec un poids moléculaire moyen Mn dans la plage de 1.000 - 50.000 g/mol, de préférence de 1.000 - 20.000 g/mol et de manière particulièrement préférée de 3.000 - 5.000 g/mol.

8. Solution de dialyse selon l'une quelconque des revendications précédentes, **caractérisée en ce que** la solution présente des électrolytes ainsi qu'un tampon.

9. Solution de dialyse selon la revendication 8, **caractérisée en ce que** les électrolytes comprennent les ions de sodium et/ou de potassium et/ou de calcium et/ou de magnésium.

10. Solution de dialyse selon la revendication 8 ou 9, **caractérisée en ce que** le tampon présente des ions de lactate et/ou des ions hydrogénocarbonate.

11. Solution de dialyse selon l'une quelconque des revendications 1 à 10 destinée à être utilisée dans un procédé de dialyse.

12. Solution de dialyse selon la revendication 11, **caractérisée en ce que** le procédé de dialyse est l'hémodialyse ou l'hémodiafiltration.

13. Solution de dialyse selon la revendication 11, **caractérisée en ce que** le procédé de dialyse est la dialyse péritonéale.
